# EUROPÄISCHE PATENTSCHRIFT

(11) **EP 0 802 906 B1**
(45) Veröffentlichungstag und Bekanntmachung des Hinweises auf die Patenterteilung: **23.11.2005**
(21) Anmeldenummer: 96900559.4
(22) Anmeldetag: 04.01.1996
(51) Int. Cl.: C07D 231/12

(54) **VERFAHREN ZUR HERSTELLUNG VON 3,5-DIARYLPYRAZOLEN**
PROCESS FOR PRODUCING 3,5-DIARYL PYRAZOLENE
PROCEDE DE PREPARATION DE 3,5-DIARYLPARAZOLENE

(30) Priorität: 13.01.1995 DE 19500838
(43) Veröffentlichungstag der Anmeldung: 29.10.1997
(73) Patentinhaber: BASF Aktiengesellschaft, 67056 Ludwigshafen (DE)
(72) Erfinder: MERKLE, Hans, Rupert, D-67061 Ludwigshafen (DE); FRETSCHNER, Erich, D-69239 Neckarsteinach (DE)
(74) Vertreter: Kinzebach, Werner
(86) Internationale Anmeldenummer: PCT/EP1996/000011
(87) Internationale Veröffentlichungsnummer: WO 1996/021650

(56) Entgegenhaltungen:
- EP-A- 0 402 722
- DE-A- 4 328 228
- US-A- 3 952 010

## Beschreibung

Die vorliegende Erfindung betrifft ein neues Verfahren zur Herstellung von 3, 5-Diarylpyrasolen der allgemeinen Formel I 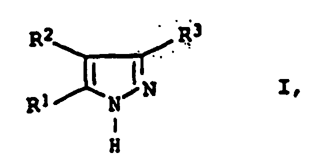 in der die Substituenten die folgende Bedeutung haben:
- R¹ und R³: Phenyl oder mit C₁-C₈-Alkyl, C₁-C₈-Alkoxy, Halogen, Nitro, Sulfonsäure, C₃-C₈-Cycloalkyl oder Allyl substituiertes Phenyl und
- R²: Wasserstoff, C₁-C₈-Alkyl, Phenyl oder mit C₁-C₈-Alkyl, C₁-C₈-Alkoxy, Halogen, Nitro, Sulfonsäure, C₃-C₈-Cycloalkyl oder Allyl substituiertes Phenyl,

3,5-Diphenylpyrazol kann durch Umsetzung von Diphenylpropandion-1,3 (Dibenzoylmethan) mit Hydrazinhydrat hergestellt werden, was beispielsweise in der US 3,882,142 beschrieben ist.

Weiterhin'führt die Umsetzung von Benzylidenacetophenondibromid mit Hydrazinhydrat zum 3,5-Diphenylpyrazol (Freudenberg, Stell, Justus Liebigs-Ann. Chem., 440 (1924) 45.

Andere Herstellungsmöglichkeiten sind Dehydrierungen von 3,5-Diphenylpyrazolinen durch beispielsweise katalytische Dehydrierung gemäß US 4,014,896, US 3,952,010 und DE 24 41 504.

Die entsprechenden Pyrazoline lassen:sich durch Umsetzung von Chalconen (1,3-Diarylpropenonen, z.B. Benzalacetophenon) in alkoholischer Lösung mit Hydrazinhydrat gewinnen (Kishner Zh. Russ. Fiz.-Khim. O-va 47 (1915) 1102; Centralblatt, 1916 I, 1063).

Eine weitere Synthesemöglichkeit für 3,5-Diphenylpyrazolin ist die Umsetzung von Phenylphenylacetylenyl-carbinol mit Hydrazinhydrat (Stoll, Justus Liebigs Anm. Chem., 440 (1924), 39, 42).

Die US 4,014,896, US 3,952,010 und DE 24' 41 504 beschreiben neben der Herstellung von Pyrazolen durch Dehydrierung der 3,5-disubstituierten Pyrazoline auch die Synthese der letzteren durch Reaktion von 1,3-disubstituierten Propenonen mit Hydrazinhydrat. Man verfährt hier im allgemeinen so, daß man ein Methylarylketon, wie z.B. Acetophenon, mit Benzaldehyd in Methanol in Anwesenheit von Basen zu den entsprechenden 1,3-disubstituierten Propenonen umsetzt, die nach Ansäuern mit Mineralsäuren mit Hydrazinhydrat zu den gewünschten Pyrazolinen abreagieren.

Aus der EP 402 722 ist ein weiteres Verfahren zur Herstellung von Pyrazol durch Dehydrierung von 2-Pyrazolin bekannt.

Da es sich bei den beschriebenen Pyrazolsynthesen um Mehrstufensynthesen handelt, stellte sich daher die Aufgabe, 3,5-Diarylpyrazole auf technisch einfachere und wirtschaftlichere Weise zugänglich zu machen.

Demgemäß wurde ein Verfahren zur Herstellung von 3,5-Diarylpyrazol der allgemeinen Formel I gefunden, 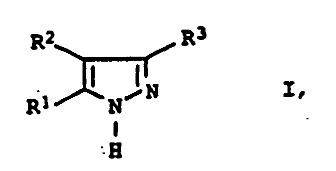 in der die Substituenten die folgende Bedeutung haben:
- R¹ und R³: Phenyl oder mit C₁-C₈-Alkyl, C₁₋₈-Alkoxy, Halogen, Nitro, Sulfonsäure, C₃-C₈-Cycloalkyl oder Allyl substituiertes Phenyl und
- R²: Wasserstoff, C₁-C₈-Alkyl, Phenyl oder mit C₁-C₈-Alkyl, C₁-C₈-Alkoxy, Halogen, Nitro, Sulfonsäure, C₃-C₈-Cycloalkyl oder Allyl substituiertes Phenyl,
dadurch gekennzeichnet, dass man Hydrazinhydrat mit
einer Carbonylverbindung der allgemeinen Formel III 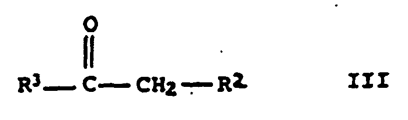 und einem Arylaldehyd der allgemeinen Formel IV

R¹-CHO IV,

in der die Substituenten R¹, R² und R³ die oben angegebene Bedeutung haben, in Schwetelsaure in Gegenwart von Jod oder einer Jodverbindung umsetzt.

Die Reaktion verläuft im Eintopfverfahren.

Das aus Hydrazinhydrat, der Carbonylverbindung der Formel III und Arylaldehyd der Formel IV gebildete 1,3-disubstituierte Pyrazolin wird im Reaktionsgemisch mit dem aus Jodwasserstoff und Schwefelsäure gebildeten Jod zum korrespondierenden 3,5-Diarylpyrazol dehydriert, wobei durch die Oxidation von Jodwasserstoff zu Jod mit Schwefelsäure entsprechende Mengen Schwefeldioxid freigesetzt werden. Die Reaktanden Hydrazinhydrat, die Carbonylverbindung der Formel III und der Arylaldehyd der Formel IV, werden in stöchiometrischen Mengen umgesetzt, wobei die Reaktanden im Überschuß oder Unterschuß eingesetzt werden können.

Die Schwefelsäure, die als Oxidationsmittel und auch als Verdünnungsmittel eingesetzt wird, wird im allgemeinen im 0,5- bis 10-fachen Üerschuß eingesetzt, wobei die Schwefelsäure in einer Konzentration von nicht weniger als 30 Gew.-%, vorzugsweise von 45 bis 95 Gew.-% vorliegen soll.

Jod oder die Jodverbindung wird bei der Umsetzung im allgemeinen in Mengen von 0,01 bis 10 Mol-% pro Mol des eingesetzten Hydrazins, vorzugsweise 0,05 bis 5 Mol-% und insbesondere 0,1 bis 2 Mol-% eingesetzt.

Neben elementarem Jod eignen sich auch Jodverbindungen wie Jodwasserstoff-, Alkalimetall- und Erdalkalimetalljodide wie Lithiumjodid, Natriumjodid, Kaliumjodid, Cäsiumjodid, Magnesiumjodid und Calziumjodid sowie sonstige Metalljodide; es können auch andere anorganische Jodverbindungen wie Erdalkalimetall- und Alkalimetallhypojodide, -jodite, -jodate und -perjodate oder organische Jodverbindungen wie Alkalijodide, z.B. Methyljodid zur Anwendung kommen.

Man führt diese Eintopfreaktion im allgemeinen bei Normaldruck bei Temperaturen von 50°C bis 250°C, vorzugsweise bei 70°C bis 200°C, insbesondere bei 90°C bis 170°C durch. Die Reaktion kann auch im Überdruck sowie im Unterdruck durchgeführt werden. Die Reaktion kann auch stufenweise durchgeführt werden, so daß man zunächst die 1,3-Diarylpropenonbildung bei 20°C bis 100°C in Schwefelsäure durchführt, wobei die für die Gesamtumsetzung benötigte Schwefelsäuremenge ganz oder zum Teil vorgelegt werden kann. Anschließend wird das Reaktionsgemisch mit Hydrazinhydrat in Anwesenheit von Jod und eventuell von zusätzlicher Schwefelsäure bei Temperaturen von 50°C bis 150°C, vorzugsweise bei 70°C bis 200°C, insbesondere bei 90°C bis 170°C umgesetzt.

Die Reaktion wird im allgemeinen so durchgeführt, daß alle Reaktionspartner im Reaktionsgefäß vereinigt und danach gemeinsam auf Reaktionstemperatur erwärmt werden. Man kann aber auch die Reaktanden, außer Schwefelsäure, getrennt oder als Mischung in vorgeheizte Schwefelsäure/NaI-Lösung eintragen oder einen Teil der Reaktanden bei Reaktionstemperatur vorlegen und die weiteren Reaktanden bei höherer Temperatur zugeben. Das Reaktionswasser wird während der Umsetzung abdestilliert.

Schon bei Reaktionstemperatur fällt das gebildete Diarylpyrazol aus und kann durch Abkühlung vollständig auskristallisiert werden. Durch Zugabe von verdünnter Natronlauge kann der Reaktionsaustrag neutralisiert werden. Auf die Neutralisation der im Reaktionsgemisch vorhandenen Schwefelsäure kann aber auch verzichtet werden, weil die 3,5-Diarylpyrazole mit Schwefelsäure unter diesen Bedingungen keine Salze bilden. Deshalb kann die Reaktionssuspension auch durch Zugabe von Wasser allein verdünnt und filtriert werden. Man erhält die Diarylpyrazole in hoher Ausbeute und Reinheit.

Das erfindungsgemäße Verfahren eignet sich zur Herstellung von 3,5-Diarylpyrazolen der allgemeinen Formel I 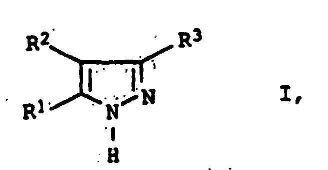 worin die Substituenten R¹, R² und R³ die vorgenannten Bedentungen aufweisen.

Im Hinblick auf das neue Verfahren wird die Reaktion in Form einer Eintopfreaktion durchgeführt, ohne eine der Zwischenstufen zu isolieren. Die Umsetzung erfolgt zweckmäßigerweise derart, daß man die Carbonylverbindungen der Formel III und Arylaldehyde der Formel IV getrennt oder als Mischung, gleichzeitig mit der stöchiometrischen Menge Hydrazinhydrat bzw. mit einem Unterschuß oder Überschuß von Hydrazinhydrat unter den vorstehend geschilderten Bedingungen in Schwefelsäure in Gegenwart von Jod bzw. einer Jodverbindung umsetzt.

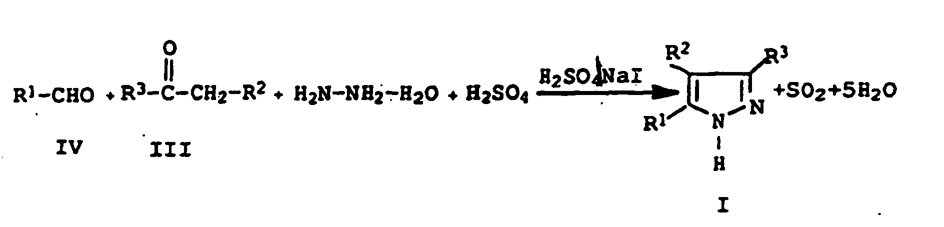

Bei dem erfindungsgemäßen Verfahren ist es von geringer Bedeutung für Ausbeute und Reinheit, in welcher Reihenfolge die Reaktanden zugegeben werden, d.h., man kann die Carbonylverbindung der Formel III und Atylaldehyd der Formel IV in Schwefelsäure vorlegen, wobei sich bereits die entsprechenden 1,3-Diarylpropenone der Formel II bilden, und Hydrazinhydrat zugeben.

Ebenso kann man Hydrazinhydrat in Schwefelsäure vorlegen und eine Mischung der Carbonylverbindung der Formel III und Arylaldehyd der Formel IV zufügen. Genauso können Mischungen von Carbonylverbindungen der Formel III und Arylaldehyd der Formel IV sowie Hydrazinhydrat getrennt, gleichzeitig oder hintereinander in die auf Reaktionstemperatur gebrachte Schwefelsäure, die-mit NaI versetzt ist, eingebracht und zur Reaktion geführt werden. Der Katalysator Jod bzw. Jodverbindung kann vorgelegt werden oder während der Reaktion zugefügt werden.

Unter den Reaktionsbedingungen werden wahrscheinlich die Zwischenstufen in situ erzeugt und reagieren nach dem folgenden Schema zu den erfindungsgemäßen Verbindungen ab.

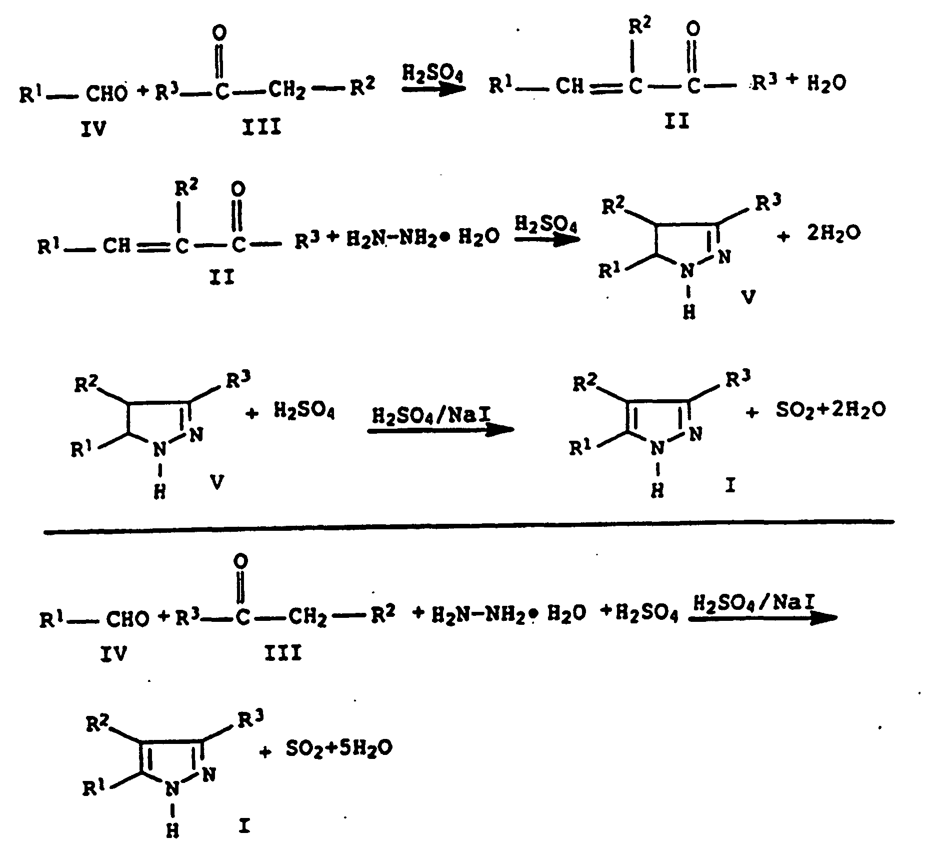

### Versuchsbeispiele:

### Beispiel 3:

392 Teile (2 mol) Schwefelsaüre 50-%ig und 1 Teil (0,0067 mol) Natriumjodid wurden im Rührkolben vorgelegt. Bei Rückfluß - 124°C wurden gleichzeitig 60 Teile (0,5 mol) Acetophenon, 53 Teile (0,5 mol) Benzaldehyd und 25 Teile (0,5 mol) Hydrazinhydrat in 30 Minuten zugetropft. Die Temperatur fiel auf 112°C. Durch Abdestillieren von Wasser wurde die Temperatur auf 130°C gebracht und bei 130°C 60 Minuten nachgerührt. 120 ml Wasser wurden abdestilliert. Das erhaltene Reaktionsgemisch wurde mit 250 ml Wasser verdünnt und mit 525 Teilen (3,28 mol) Natronlauge 25 gew.-%ig neutralisiert und filtriert. Der Filterrückstand wurde mit Wasser sulfatfrei gewaschen und getrocknet. Man erhielt 110,2 Teile 3,5-Diphenylpyrazol mit einem Gehalt von 94,1 % (HPLC), das entspricht einer Ausbeute von 94,3 % d. Theorie. Fp: 194°C.

### Beispiel 4:

25 Teile (0,5 mol) Hydrazinhydrat und 60 Teile (0,5 mol) Acetophenon wurden gleichzeitig in vorgelegte 326,7 Teile Schwefelsäure 60 gew.-%ig eingetropft. Bei 120°C wurde 2 Stunden nachgerührt und anschließend auf 50°C abgekühlt. In das Reaktionsgemisch wurden 53 Teile (0,5 mol) Benzaldehyd eingetropft und erneut 2 Stunden bei 120°C nachgerührt. 5 Teile (0,033 mol) Natriumjodid 10 gew.-%ig wurden in 10 Minuten zugetropft und der Ansatz 4,5 Stunden bei Rückfluß nachgerührt. Nach dem Abkühlen und Verdünnen mit 100 Teilen Wasser wurde das Reaktionsprodukt abfiltriert und mit Wasser neutral gewaschen. Man erhielt 111,2 Teile 3,5-Diphenylpyrazol mit einem Gehalt von 92,7 % (HPLC), das entspricht einer Ausbeute von 93,7 % d. Theorie. Fp: 192°C.

### Beispiel 6:

60 Teile (0,5 mol) Acetophenon und 53 Teile (0,5 mol) Benzaldehyd wurden gleichzeitig bei 25°C in vorgelegte 140 Teile (1 mol) Schwefelsäure 70 gew.-%ig eingetropft. Bei 25°C wurde 60 Minuten nachgerührt und die erhaltene Reaktionslösung gleichzeitig mit 25- Teilen (0, 5 mol) Hydrazinhydrat bei 135°C in vorgelegte 140 Teile (1 mol) Schwefelsäure 70 gew.-%ig und 0,5 Teile (0,0033 mol) Natriümjodid in 25 Minuten eingetropft. Bei 125°C wurde 60 Minuten nachgerührt. Um die Temperatur bei 135°C zu halten, wurden 38 Teile Wasser abdestilliert. Der Ansatz wurde mit 500 Teilen Wasser verdünnt und mit 247,8 Teilen (3,1 mol) Natronlauge 50 gew.-%ig neutralisiert. Nach dem Abfiltrieren des Feststoffs wurde sulfatfrei gewaschen und getrocknet. Man erhielt 109, 7 Teile 3,5-Diphenylpyrazol mit einem Gehalt von 96,8 % (HPLC), das entspricht einer Ausbeute von 96,5 % d. Theorie. Fp: 197°C.

### Beispiel 7:

490 Teile (3 mol) Schwefelsäure 60 gew.-%ig und 1,5 Teile (0,01 mol) Natriumjodid wurden im Rührkolben vorgelegt. Bei 120°C wurden gleichzeitig 46,9 Teile (0,75 mol) Hydrazinhydrat 80 gew.-%ig, 90 Teile (0.7-5 mol) Acetophenon und 79,5 Teile (0,75 mol) Benzaldehyd in 2 Stunden zugetropft. Durch Abdestillieren von 120 Teilen Wasser wurde die Temperatur 2 Stunden bei 120°C gehalten. Das erhaltene Reaktionsgemisch wurde mit dem abdestillierten Wasser verdünnt und mit 936 Teilen (4,7 mol) Natronlauge 20 gew.-%ig neutralisiert. Bei Raumtemperatur wurde abfiltriert, der Filterrückstand mit Wasser gewaschen und getrocknet. Man erhielt 162,7 Teile 3,5-Diphenylpyrazol mit einem Gehalt von 96,1 % (HPLC), das entspricht einer Ausbeute von 94,8 % d. Theorie. Fp: 196°C.

### Beispiel 8:

490 Teile (3 mol) Schwefelsäure 60 gew.-%ig wurden im Rührkolben vorgelegt. Bei Raumtemperatur wurden nacheinander 46,9 Teile (0,75 mol) Hydrazinhydrat 80 gew.-%ig, 90 Teile (0,75 mol) Acetophenon, 79,5 Teile (0,75 mol) Benzaldehyd und 1,5 Teile (0,01 mol) Natriumjodid zudosiert. Das Reaktionsgemisch wurde durch Abdestillieren von Wasser auf 120°C gebracht und 2,5 Stunden bei 120°C gehalten. Es wurden 110 Teile Wasser abdestilliert. Der Ansatz wurde mit dem abdestillierten Wasser verdünnt und mit 717 Teilen (4,5 mol) Natronlauge 25 gew.-%ig neutralisiert. Bei Raumtemperatur wurde abfiltriert, der Filterrückstand mit Wasser gewaschen und getrocknet. Man erhielt 160,6 Teile 3,5-Diphenylpyrazol mit einem Gehalt von 94,4 % (HPLC), das entspricht einer Ausbeute von 91,9 % d. Theorie. Fp: 194°C.

### Beispiel 10:

326,7 Teile (2 mol) Schwefelsäure wurden im Rührkolben vorgelegt. Bei Raumtemperatur wurden nacheinander 37,5 Teile (0,75 mol) Hydrazinhydrat, 100 Teile (0,75 mol) 4-Methylacetophenon, 105 Teile (0,75 mol) 4-Chlorbenzaldehyd und 1 Teil (0,0067 mol) Natriumjodid zudosiert. Das Reaktionsgemisch wurde durch Abdestillieren von Wasser auf 140°C gebracht und 2,5 Stunden bei 140°C gehalten. Es wurden 140 Teile Wasser abdestilliert. Der Ansatz wurde mit 500 Teilen Wasser verdünnt und mit 500 Teilen (12,5 mol) Natronlauge 20 gew.-%ig neutralisiert. Nach dem Abfiltrieren des Feststoffs wurde sulfatfrei gewaschen und getrocknet. Man erhielt 189 Teile 3-(4-Chlorphenyl)-5-(4-methylphenyl)-pyrazol mit einem Gehalt von 98 % (HPLC), das entspricht einer Ausbeute von 92 % d. Theorie. Fp: 228°C.

## Patentansprüche

1. Verfahren zur Herstellung von 3,5-Diarylpyrazolen der allgemeinen Formel I 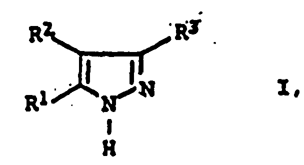 in der die Substituenten die folgende Bedeutung haben:
R¹ und R³ Phenyl oder mit C₁-C₈-Alkyl, C₁-C₈-Alkoxy, Halogen, Nitro, Sulfonsäure, C₃-C₈-Cycloalkyl oder Allyl substituiertes Phenyl und
R² Wasserstoff, C₁-C₈-Alkyl, Phenyl oder mit C₁-C₈-Alkyl, C₁-C₈-Alkoxy, Halogen, Nitro, Sulfonsäure, C₃-C₈-Cycloalkyl oder Allyl substituiertes Phenyl,
**dadurch gekennzeichnet, dass** man Hydrazinhydrat mit
einer Carbonylverbindung der allgemeinen Formel III 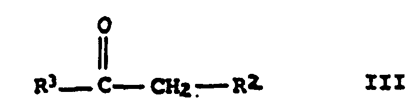 und einem Arylaldehyd der allgemeinen Formel IV
R¹-CHO IV.
in der die Substituenten R¹, R² und R³ die oben angegebene Bedeutung haben, in Schwefelsäure in Gegenwart von Jod oder einer Jodverbindung umsetzt.

2. Verfahren nach Anspruch 1, **dadurch gekennzeichnet, dass** man die Umsetzung bel einer Temperatur von 50 bis 250°C durchführt.

3. Verfahren gemäß einem der Ansprüche 1 oder 2, **dadurch gekennzeichnet, daß** man die Umsetzung in Gegenwart, von 0,01 bis 10 mol-% Jod oder einer Jodverbindung, bezogen auf das eingesetzte Hydrazin, durchführt.

4. Verfahren gemäß einem der Ansprüche 1 bis 3, **dadurch gekennzeichnet, daß** man die Umsetzung in 0,5- bis 10-fachem Überschuß Schwefelsäure durchführt, bezogen auf die Ausgangskomponenten der Formeln III und IV.

## Claims

1. A process for preparing 3,5-diarylpyrazoles of the general formula I 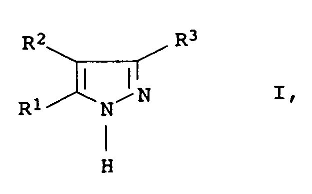 where the substituents have the following meanings:
R¹ and R³ are phenyl or phenyl substituted by C₁-C₈-alkyl, C₁-C₈-alkoxy, halogen, nitro, sulfonic acid, C₃-C₈-cycloalkyl or allyl and
R² is hydrogen C₁-C₈-alkyl, phenyl or phenyl substituted by C₁-C₈-alkyl, C₁-C₈-alkoxy, halogen, nitro, sulfonic acid, C₃-C₈-cycloalkyl or allyl,
which comprises reacting hydrazine hydrate with a carbonyl compound of the general formula III 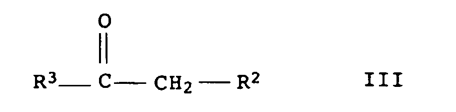 and an arylaldehyde of the general formula IV
R¹-CHO IV,
where the substituents R¹, R² and R³ have the abovementioned meanings, in sulfuric acid in the presence of iodine or an iodine compound.

2. The process according to claim 1, wherein the reaction is carried out at from 50 to 250°C.

3. The process according to one of claims 1 and 2 wherein the reaction is carried out in the presence of from 0.01 to 10 mol% of iodine or an iodine compound, based on the hydrazine employed.

4. The process according to one of claims 1 to 3 wherein the reaction is carried out in a from 0.5- to 10-fold excess of sulfuric acid, based on the starting components of the formulae III and IV.

## Revendications

1. Procédé de préparation de 3,5-diarylpyrazoles de la formule générale I : 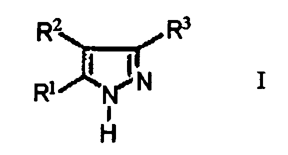 dans laquelle les substituants ont la signification suivante :
R¹ et R³ représentent un radical phényle ou phényle substitué par de l'halogène, de l'acide sulfonique ou un groupe alkyle en C₁-C₈, alcoxy en C₁-C₈, nitro, cycloalkyle en C₃-C₈ ou allyle, et
R² représente de l'hydrogène ou un radical alkyle en C₁-C₈, phényle ou phényle substitué par de l'halogène, de l'acide sulfonique ou un groupe alkyle en C₁ -C₈, alcoxy en C₁-C₈, nitro, cycloalkyle en C₃-C₈ ou allyle,
**caractérisé en ce qu'**on fait réagir de l'hydrate d'hydrazine avec un composé carbonyle de la formule générale III: 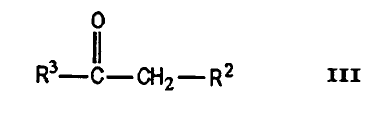 et un aldéhyde arylique de la formule générale IV :
R¹ - CHO IV
où les substituants R¹, R² et R³ ont les significations indiquées ci-dessus, dans de l'acide sulfurique, en présence d'iode ou d'un composé d'iode.

2. Procédé suivant la revendication 1, **caractérisé en ce qu'**on effectue la réaction à une température de 50 à 250°C.

3. Procédé suivant l'une des revendications 1 et 2, **caractérisé en ce qu'**on effectue la réaction en présence de 0,01 à 10 % molaires d'iode ou d'un composé d'iode, par rapport à l'hydrazine mise en oeuvre.

4. Procédé suivant l'une des revendications 1 à 3, **caractérisé en ce qu'**on effectue la réaction dans un excès d'acide sulfurique de 0,5 à 10 fois par rapport aux composants de départ des formules III et IV.
